# EUROPÄISCHE PATENTSCHRIFT

(11) **EP 2 510 978 B1**
(45) Veröffentlichungstag und Bekanntmachung des Hinweises auf die Patenterteilung: **14.05.2014**
(21) Anmeldenummer: 12156706.9
(22) Anmeldetag: 23.02.2012
(51) Int. Cl.: A61N 5/10

(54) **Verfahren zur Bestrahlungsplanung und Bestrahlungsplanungseinrichtung**
Method for irradiation planning and irradiation planning device
Procédé de planification d'irradiation et dispositif de planification d'irradiation

(30) Priorität: 11.04.2011 DE 102011007148
(43) Veröffentlichungstag der Anmeldung: 17.10.2012
(73) Patentinhaber: Siemens Aktiengesellschaft, 80333 München (DE)
(72) Erfinder: Gemmel, Alexander, Dr., 91054 Erlangen (DE); Rietzel, Eike, Dr., 64331 Weiterstadt (DE)

(56) Entgegenhaltungen:
- EP-A2- 1 905 481
- EP-A2- 2 279 776
- DE-A1-102008 009 765
- KRAEMER M ET AL: "TREATMENT PLANNING FOR HEAVY-IRON RADIOTHERAPY: PHYSICAL BEAM MODEL AND DOSE OPTIMIZATION", PHYSICS IN MEDICINE AND BIOLOGY, TAYLOR AND FRANCIS LTD. LONDON, GB, Bd. 45, Nr. 11, 1. November 2000 (2000-11-01), Seiten 3299-3317, XP001146537, ISSN: 0031-9155, DOI: 10.1088/0031-9155/45/11/313
- SCHELL STEFAN ET AL: "Advanced treatment planning methods for efficient radiation therapy with laser accelerated proton and ion beams", MEDICAL PHYSICS, AIP, MELVILLE, NY, US, Bd. 37, Nr. 10, 20. September 2010 (2010-09-20), Seiten 5330-5340, XP012144701, ISSN: 0094-2405, DOI: 10.1118/1.3491406

## Beschreibung

Die Erfindung betrifft ein Planungsverfahren für ein Partikeltherapie-Bestrahlungsverfahren, bei dem eine zu deponierende Dosis Isoenergieschicht-weise im zu bestrahlenden Zielvolumen appliziert wird. Weiterhin betrifft die Erfindung eine entsprechende Bestrahlungsplanungseinrichtung.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Teilchenstrahl geformt und über ein Hochenergiestrahltransportsystem zu einem oder mehreren Bestrahlungsräumen geführt. In einem dieser Bestrahlungsräume wird das zu bestrahlende Objekt mit einem Zielvolumen mit dem Teilchenstrahl bestrahlt.

Eine Bestrahlung mit Partikeln kann Isoenergieschicht-weise durchgeführt werden. Dabei wird das zu bestrahlende Zielvolumen in eine Vielzahl von in Strahlrichtung hintereinander liegenden Isoenergieschichten aufgeteilt. Jede Isoenergieschicht ist mit einer bestimmten Partikelenergie assoziiert, die durch die Eindringtiefe der Partikel mit dieser Energie in das zu bestrahlende Zielobjekt charakterisiert ist.

Bei der Bestrahlung wird dann die Energie der Partikel entsprechend eingestellt, um die zugehörige Isoenergieschicht mit einer gewünschten Dosis zu bestrahlen. Nachdem diese Isoenergieschicht bestrahlt worden ist, wird die Energie der Partikel verändert, um die nächste Isoenergieschicht zu bestrahlen. Dieses Verfahren wird so lange wiederholt, bis alle Isoenergieschichten bestrahlt worden sind und das Zielvolumen dadurch vollständig mit der gewünschten Dosis bestrahlt worden ist.

Innerhalb einer Isoenergieschicht kann die Dosis dann auf verschiedene Weisen deponiert werden. Beispielsweise kann ein Scanverfahren zur Anwendung kommen, bei dem der Partikelstrahl sukzessive an verschiedene Orte innerhalb der Isoenergieschicht gelenkt wird, um dort jeweils eine gewünschte Dosis zu abzugeben.

Eine Isoenergieschicht-weise Bestrahlung ist z.B. in der DE 10 2008 009 765 A1 beschrieben.

In diesem Dokument definiert ein Datensatz Zielpunkte in einem Zielvolumen in einem Körper, auf die in einem kontinuierlichen oder diskontinuierlichen Prozess nacheinander ein Partikelstrahl zu richten ist, wobei durch Richten des Partikelstrahls auf einen Zielpunkt in einer Umgebung des Zielpunktes eine vorbestimmte räumliche Dosisverteilung zu erzeugen ist und wobei jeder Zielpunkt einen in einem homogenen Körper, der zu dem Körper äquivalent ist, in Richtung des Partikelstrahls gemessenen z-Abstand von einem nächst benachbarten Zielpunkt bei einer höheren oder niedrigeren Partikelenergie aufweist. Bei einem Verfahren zum Erzeugen des Datensatzes werden die Zielpunkte in dem Datensatz so definiert, dass zumindest entweder die z-Abstände oder die räumlichen Dosisverteilungen von der jeweiligen Partikelenergie abhängen.

Es ist die Aufgabe der Erfindung, ein Planungsverfahren für ein Isoenergieschicht-weises Partikeltherapie-Bestrahlungsverfahren anzugeben, das es ermöglicht, ein Zielvolumen mit einer Solldosis zu bestrahlen und dabei gleichzeitig das Gewebe, das das Zielvolumen umgibt, schont. Weiterhin betrifft die Erfindung eine Bestrahlungsplanungseinrichtung, auf der ein derartiges Planungsverfahren ausführbar ist.

Die Erfindung wird durch die unabhängigen Ansprüche gelöst.

Das Verfahren zur Bestrahlungsplanung für ein Partikeltherapie-Bestrahlungsverfahren, in der eine Dosis entlang mehrerer in Strahlrichtung hintereinander angeordneten Isoenergieschichten zu applizieren ist, umfasst folgende Schritte:
- Vorgeben eines zu bestrahlenden Zielvolumens,
- Bestimmen einer ersten Position einer proximalsten Isoenergieschicht in Bezug auf das zu bestrahlende Zielvolumen,
- Bestimmen einer zweiten Position zumindest einer weiteren Isoenergieschicht, die distal zu der proximalsten Isoenergieschicht liegt, in Bezug auf das zu bestrahlende Zielvolumen unter Verwendung der ersten Position.

Die Bezeichnungen distal und proximal beziehen sich dabei auf die Lage der Isoenergieschichten in Bezug auf die Strahlrichtung. "Proximal" bedeutet in Strahlrichtung gesehen vorne gelegen und ist mit einer geringen Energie der Partikel bzw. mit einer geringen Eindringtiefe verbunden. "Distal" bedeutet in Strahlrichtung gesehen hinten liegend, und ist mit einer hohen Energie der Partikel bzw. mit einer großen Eindringtiefe verbunden.

Die Erfindung beruht auf der Erkenntnis, dass bisherige Planungsverfahren für eine Isoenergieschicht-weise Bestrahlung mit Nachteilen behaftet sind. So werden bei bisherigen Planungsverfahren Isoenergieschichten mit einem festen Abstand im Zielvolumen positioniert. Dies geschieht überlicherweise derart, dass die distalste Isoenergieschicht so platziert wird, dass das Zielvolumen gerade noch überdeckt wird. Die Eindringtiefe der Partikel, die zur distalsten Isoenergieschicht gehören, ist damit passend zur distalen Kante des Zielvolumens gewählt.

Die Lage der anderen Isoenergieschichten ist dann unabhängig von der Tumorform durch den festen Abstand der Isoenergieschichten zueinander gegeben. Je nach Form des Zielvolumens und je nach Abstand der Isoenergieschichten kann es dann geschehen, dass aufgrund der Lage der proximalsten Isoenergieschicht das Gebiet, das in Strahlrichtung vor dem Zielvolumen liegt, mit einer erheblichen Dosis belastet wird.

Es wurde erkannt, dass sich dieser Effekt umso größer auswirkt, je breiter der Bragg-Peak des Partikelstrahls gewählt wird. Dies geht nämlich mit einer größeren Breite der Isoenergieschichten einher. Dadurch wird der Abstand von Isoenergieschicht zu Isoenergieschicht ebenfalls größer. Die Wahrscheinlichkeit nimmt dann zu, das die proximalste Isoenergieschicht in das vor dem Zielvolumen liegende Gebiet in nicht unerheblicher Weise hineinragt.

Mögliche Lösungen für dieses Problem, die auf einer Verkleinerung des Bragg-Peaks (oder auf einer Verkleinerung des Abstandes der Isoenergieschichten) beruhen, haben zur Folge, dass eine größere Zahl an Isoenergieschichten notwendig ist, um das Zielvolumen zu überdecken. Eine darauf folgende Bestrahlung würde dann aber zu einer Verlängerung der Bestrahlungsdauer führen.

Andere Lösungen, die einen Bolus verwenden, d.h. eine Material-Platte, um zusätzliche - gegebenenfalls Zielvolumen spezifische - Änderungen der gewählten Energie zu erzwingen, um die Dosisdeposition an genauer an das Zielvolumen anzupassen, erhöhen den Aufwand der Bestrahlung und bewirken zusätzliche negative Effekte aufgrund von Strahlstreuung und Strahlfragmentierung.

Bei dem hier vorgestellten Planungsverfahren wird nun zunächst die proximalste Isoenergieschicht betrachtet. Deren Lage kann so gewählt werden, dass möglichst wenig Hochdosis im Gebiet vor dem eigentlichen Zielvolumen deponiert wird. Die Lage kann so gewählt werden, dass bei einer Bestrahlung der proximalsten Isoenergieschicht der Teil der dabei deponierten Dosis, der im Zielvolumen zu liegen kommt, größer ist als der Teil der deponierten Dosis, der in Strahlrichtung gesehen vor dem Zielvolumen liegt. Der im Zielvolumen liegende Teil der Dosis kann insbesondere doppelt so groß oder gar noch größer sein als der Teil, der vor dem Zielvolumen zu liegen kommt. Anschließend werden eine oder mehrere weiter distal gelegenen Isoenergieschichten (bzw. deren Lage) im Zielvolumen bestimmt. Die Bestimmung der Lage der weiteren, weiter distal gelegenen Isoenergieschichten, also z.B. deren Lage im Zielvolumen und deren Abstand zueinander, kann derart erfolgen, dass eine gewünschte Solldosisverteilung im Zielvolumen durch die weitere Bestrahlungsplanung erreicht werden kann bzw. auch erreicht wird.

Nachdem die Isoenergieschichten im Zielvolumen positioniert sind, können z.B. Rasterpunkte in den Isoenergieschichten definiert werden, für welche dann im Laufe der Bestrahlungsplanung festgelegt wird, welche Dosis den Rasterpunkten zugeordnet wird.

Letztlich wird auf diese Weise erreicht, dass die zu deponierende Dosis möglichst zielgenau im Zielvolumen abgegeben wird, selbst wenn man die Bestrahlung mit einem ausgeweiteten Bragg-Peak plant.

Das Bestimmen der Position einer Isoenergieschicht in Bezug auf das Zielvolumen kann dadurch erfolgen, dass die Lage der Isoenergieschicht im Zielvolumen zunächst durch rein geometrische Betrachtung des Zielvolumens und der Isoenergieschicht bestimmt wird. Z.B. kann die Isoenergieschicht durch einen geometrischen Parameter gekennzeichnet wird, und so der Bezug zwischen der Position der Isoenergieschicht und dem Zielvolumen hergestellt wird. Anhand dieser geometrisch festgelegten Position kann dann die Energie der Teilchen bestimmt werden, die mit dieser Position im Zielvolumen assoziiert ist.

Das Bestimmen der Position einer Isoenergieschicht in Bezug auf das Zielvolumen kann aber auch dadurch erfolgen, dass die Position nicht geometrisch, sondern indirekt über die Energie einer Isoenergieschicht bestimmt wird. Da die Position der Isoenergieschicht im Zielvolumen mit der Energie der Isoenergieschicht fest korreliert, kann die Position einer Isoenergieschicht in Bezug auf das Zielvolumen auch über dessen Energie festgelegt werden, ohne zunächst die Lage geometrisch zu bestimmen. Auf diese Weise kann besser auf die Randbedingungen eines Beschleunigers Rücksicht genommen werden. Wenn ein Beschleuniger es z.B. nur erlaubt, die Partikelenergie in diskreten Stufen zu verändern, können von vornherein nur diejenigen Positionen im Zielvolumen zugelassen, die den disketen Energiestufen entsprechen. Durch Wahl der am besten passenden Energiestufe kann dann die Position der proximalsten Isoenergieschicht festgelegt werden.

Bei dem Verfahren ist letztlich der Rasterabstand flexibel und kann der Geometrie des Zielvolumens besser angepasst werden, insbesondere besser als bei einem starren Schema. Die Berücksichtigung der proximalen Geometrie des Zielvolumens wird bei der Wahl der Isoenergieschichten berücksichtigt. Damit kann die Lage der proximalen Isoenergieschicht an das Zielvolumen optimal angepasst werden. Hochdosisbelegungen im Normalgewebe werden vermindert.

In einer Weiterbildung liegt die zumindest eine weitere Isoenergieschicht, deren Position in Abhängigkeit der ersten Position geplant wird, zwischen der proximalsten Isoenergieschicht und einer distalsten Isoenergieschicht. Die zweite Position wird dann dadurch bestimmt, dass eine dritte Position der distalsten Isoenergieschicht in Bezug auf das zu bestrahlende Zielvolumen bestimmt wird, und dass die zweite Position unter Verwendung der dritten Position erfolgt.

Bei dieser Weiterbildung werden folglich zunächst die Positionen der proximalsten und der distalsten Isoenergieschicht festgelegt. In Abhängigkeit davon erfolgt die Bestimmung der Position einer weiteren Isoenergieschicht, die zwischen diesen beiden Isoenergieschichten liegt. Mit dieser Ausgestaltung kann auf einfache Weise sichergestellt werden, dass auch die distale Isoenergieschicht passend auf die distale Kante des Zielvolumens gelegt wird. Auch hier kann die Bestimmung der Lage der weiteren Isoenergieschichten, also z.B. deren Lage im Ziel volumen und deren Abstand zueinander, derart erfolgen, dass eine gewünschte Solldosisverteilung im Zielvolumen durch die weitere Bestrahlungsplanung ermöglicht wird.

Beim Bestimmen einer Position einer der Isoenergieschichten kann in einer Ausführungsvariante ein Hochdosisbereich bestimmt werden, der den zur Isoenergieschicht zugeordneten Bereich mit einer bestimmten Dosisbelegung kennzeichnet, und dass das Bestimmen der Position der Isoenergieschicht unter Verwendung des dieser Isoenergieschicht zugeordneten Hochdosisbereichs erfolgt.

Der Hochdosisbereich kann beispielsweise den Bereich einer Isoenergieschicht kennzeichnen, in dem z.B. die Dosiskurve über einem bestimmten Prozentwert des Bragg-Peak-Maximums liegt. Der Hochdosisbereich kennzeichnet demzufolge die Ausdehnung bzw. die Dicke der Isoenergieschicht in Strahlrichtung. Die Positionierung einer Isoenergieschicht kann verbessert und genauer an das Zielvolumen angepasst werden, wenn bei der Positionierung auch der Hochdosisbereich bzw. die Ausdehnung der Isoenergieschicht in Strahlrichtung betrachtet wird.

Der Hochdosisbereich einer Schicht hängt dabei von der Energieverbreiterung (engl.: "Energy Straggling") der verwendeten Ionensorte, der Anfangsenergie sowie z.B. von der Dicke eines zu verwendenden Ripple-Filters ab. Insbesondere bei hoher Energieverbreiterung und/oder großen Ripple-Filtern mit großem Hochdosisbereich lassen sich mit dem Bestrahlungsplanungverfahren relevante Einsparungen in der Normalgewebsdosis verwirklichen bzw. eine Erhöhung der Normalgewebsdosis beim Übergang von kleinen zu großen Ripple-Filtern vermeiden.

Insbesondere kann der Hochdosisbereich bzw. dessen Ausdehnung bestimmt werden, und zwar in Abhängigkeit von der verwendeten Partikelsorte, von der Anfangsenergie der verwendeten Partikel und/oder Einstellungen der Partikeltherapieanlage, die bei der geplanten Bestrahlung zu verwenden sind. Derartige Einstellungen können z.B. Eigenschaften einer Bragg-Peak-Aufweitungsvorrichtung wie z.B. ein Ripple-Filter sein, die letztlich einen Einfluss darauf hat, wie groß der Hochdosisbereich der Schicht ist.

Die Bestimmung kann z.B. erfolgen, indem ein in einer Tabelle oder Datenbank hinterlegter Wert verwendet wird, der einer Partikelsorte, einer Partikelenergie und/oder einem zu verwendenden Ripple-Filter hinterlegt ist.

In einer Ausführungsform kann beim Bestimmen der ersten Position der proximalsten Isoenergieschicht der zugeordnete Hochdosisbereich derart platziert werden, dass die proximale Kante des Hochdosisbereichs näher an der proximalen Kante bzw. an der proximalsten Stelle des Zielvolumens liegt als die distale Kante des Hochdosisbereichs. Auf diese Weise wird sichergestellt, dass der Großteil der zur proximalen Isoenergieschicht gehörigen Hochdosis im Zielvolumen appliziert werden kann und nicht vor dem Zielvolumen.

Ebenso kann beim Bestimmen der dritten Position der distalsten Isoenergieschicht der zugeordnete Hochdosisbereich derart platziert werden, dass die distale Kante des Hochdosisbereichs näher an der distalen Kante bzw an der distalsten Stelle des Zielvolumens liegt als die proximale Kante des Hochdosisbereichs. Auf diese Weise wird sichergestellt, dass der Großteil der zur distalen Isoenergieschicht gehörigen Hochdosis im Zielvolumen appliziert werden kann und nicht hinter dem Zielvolumen.

Die hinter der proximalsten Isoenergieschicht liegenden Isoenergieschichten können äquidistant verteilt werden oder aber auch mit variablem Abstand, wie in der DE 10 2008 009 765 A1 beschrieben.

In einer Ausgestaltung des Verfahrens kann eine Umrechnung des Zielvolumens in einen wasseräquivalenten Körper erfolgen. Das Bestimmen der Position der Isoenergieschichten in Bezug auf das Zielvolumen kann dann unter Verwendung des umgerechneten Zielvolumens erfolgen.

Bei dieser Ausführungsform erfolgt damit zumindest teilweise unter Zuhilfenahme eines wasseräquivalenten Körpers. Dies bringt Vorteile bei der weiteren Positionsbestimmung der Isoenergieschichtlage mit sich, da in einen wasseräquivalenten Körper Isoenergieschichten eine ebene Form aufweisen und da der Hochdosisbereich der Isoenergieschichten eine einfache geometrische Form wie bspw. die eines Rechtecks aufweist. Abstände zu proximalen oder distalen Kanten des Zielvolumens lassen sich besonders einfach berechnen.

Die Bestrahlungsplanungseinrichtung zur Bestrahlungsplanung für ein Partikeltherapie-Bestrahlungsverfahren, in der eine Dosis entlang mehrerer in Strahlrichtung hintereinander angeordneten Isoenergieschichten zu applizieren ist, umfasst:
a) eine Eingabeeinrichtung zum Vorgeben eines zu bestrahlenden Zielvolumens,
b) eine Schichtpositionierungseinrichtung
   - zum Bestimmen einer ersten Position einer proximalsten Isoenergieschicht in Bezug auf das zu bestrahlende Zielvolumen, und
   - zum Bestimmen einer zweiten Position zumindest einer weiteren Isoenergieschicht, die distal zu der proximalsten Isoenergieschicht liegt, in Bezug auf das zu bestrahlende Zielvolumen unter Verwendung der ersten Position.

Die Bestrahlungsplanungseinrichtung kann zur Durchführung eines Verfahrens nach einem der Verfahrensansprüche ausgebildet sein, beispielsweise durch entsprechende Ausbildung der Schichtpositionierungseinrichtung.

Die vorangehende und die folgende Beschreibung der einzelnen Merkmale, deren Vorteile und deren Wirkungen bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung werden anhand der folgenden Zeichnungen näher erläutert, ohne jedoch darauf beschränkt zu sein. Es zeigen:
- Fig. 1: die Schichtpositionierung in einem wasseräquivalenten Zielvolumen nach dem Stand der Technik,
- Fig. 2: die Schichtpositionierung in einem wasseräquivalenten Zielvolumen nach einer Ausführungsform der Erfindung,
- Fig. 3: die Schichtpositionierung in einem wasseräquivalenten Zielvolumen nach einer weiteren Ausführungsform der Erfindung,
- Fig. 4: eine Dosisverteilung an einem Patientenbeispiel mit einer Schichtpositionierung mit starrem Rasterabstand,
- Fig. 5: dasselbe Patientenbeispiel mit einer Schichtpositionierung, bei der die proximale Isoenergieschicht an das Zielvolumen adaptiert ist,
- Fig. 6: eine schematische Darstellung von Verfahrensschritten einer Ausführungsform des Bestrahlungsplanungsverfahrens, und
- Fig. 7: eine Darstellung einer Bestrahlungsplanungseinrichtung, mit der die Schichtpositionierung durchgeführt werden kann.

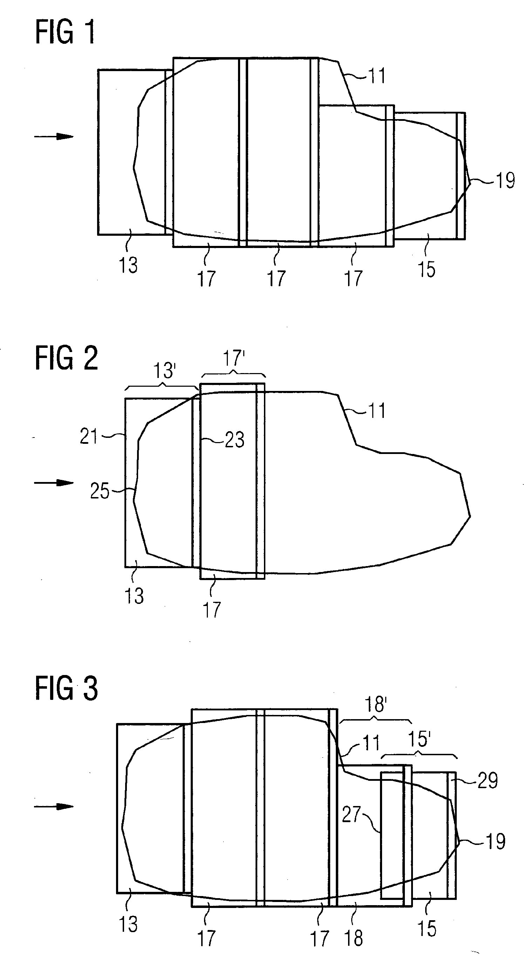

Fig. 1 zeigt ein Zielvolumen 11, das sich in einem Zielobjekt (hier nicht dargestellt) befindet und das bei der Bestrahlung mit einer möglichst homogenen Solldosis bestrahlt werden soll. Das angrenzende Gebiet, das das Zielvolumen 11 umgibt - üblicherweise ein zu schonendes Gewebe - soll mit möglichst wenig Dosis beaufschlagt werden.

Das hier dargestellte Zielvolumen 11 wurde bereits in einen wasseräquivalenten Körper umgerechnet. Dies bedeutet, dass jedes Voxel des umgerechneten Zielobjekts 11 den eintreffenden Partikelstrahl gleich stark abbremst wie ein Voxel aus Wasser. Details zur Umrechnung eines realen Zielobjekts bzw. Zielvolumens in einen wasseräquivalentes Zielobjekt bzw. Zielvolumens finden sich z.B. in der Schrift Krämer M et al, "Treatment planning for heavy-ion radiotherapy: physical beam model and dose optimization" Phys. Med. Biol. 45 (2000) 3299-3317.

In Fig. 1 ist die Bestrahlungsplanung nach dem Stand der Technik dargestellt.

Das Zielvolumen 11 wird in Isoenergieschichten 13, 15, 17 gleicher Reichweite eingeteilt. Der Pfeil zeigt die Einfallsrichtung des Partikelstrahls an. Diese Isoenergieschichten 13, 15, 17 werden mit einem festen Abstand über das Zielvolumen 11 gesetzt. Bei einer Bestrahlung mit Kohlenstoff-Ionen kann z.B. ein Schichtabstand von 3 mm verwendet werden. Die distale Isoenergieschicht 15 ist dabei derart gewählt, dass der Bragg-Peak dieser Schicht die distale Kante 19 des Zielvolumens 11 gerade noch überdeckt. Der Abfall der Dosis nach einem Bragg-Peak ist sehr steil. Dadurch ist gewährleistet, dass das Gebiet distal zum Zielvolumen 11 mit nur sehr wenig Dosis belastet wird.

Die Aufteilung der Isoenergieschichten 13, 15, 17 auf das Zielvolumen 11 nach diesem Schema ist vergleichsweise starr. Je nach Form des Zielvolumens 11 kann es zu Unter- oder Überdosierungen kommen. Außerdem kann das Hochdosisgebiet vergleichsweise stark in das vor dem Zielvolumen liegende Gebiet, das es üblicherweise zu schonen gilt, hineinreichen.

Fig. 2 zeigt ein Ausführungsbeispiel gemäß der Erfindung, das eine verbesserte Schichtaufteilung aufweist. Dabei ist hier die Positionierung der proximalsten Isoenergieschicht 13 erläutert, die bei dem Verfahren zuerst gewählt wird.

Idealerweise wird die proximalste Isoenergieschicht 13 derart platziert, dass der Hochdosisbereich 13' dieser Isoenergieschicht 13 das Zielvolumen 11 gerade noch bedeckt, indem beispielsweise die proximale Kante 23 des Hochdosisbereichs 13' an der proximalen Kante 25 des Zielvolumens 11 bzw. am proximalsten Punkt des Zielvolumens 11 liegt. Dies ist jedoch nicht immer möglich, da beispielsweise aufgrund von Einschränkungen der Partikeltherapieanlage die proximale Isoenergieschicht 13 nicht beliebig fein positioniert werden kann.

Bei der Platzierung der proximalsten Isoenergieschicht 13 kann dann darauf geachtet werden, dass die proximale Seite 21 des Hochdosisbereichs 13' näher an der proximalen Kante 25 bzw. am proximalsten Punkt des Zielvolumens 11 liegt als die distale Seite 23 des Hochdosisbereichs 13'. Auf diese Weise wird gewährleistet, dass bei dieser Isoenergieschicht 13 mehr Dosis im Zielvolumen 11 appliziert werden kann als im Gebiet vor dem Zielvolumen 11.

Nachdem die proximalste Isoenergieschicht 13 positioniert worden ist, können die anschließenden, distaleren Isoenergieschichten positioniert werden, sodass das Zielvolumen 11 dann mit Isoenergieschichten vollständig überdeckt ist.

In Fig. 2 gezeigt ist die Positionierung einer weiter distal gelegenen Isoenergieschicht 17. Die Lage dieser Isoenergieschicht 17 kann in Abhängigkeit der Lage der proximalsten Isoenergieschicht 13 bestimmt werden. So kann z.B. die zweitproximalste Isoenergieschicht 17 so gelegt werden, dass ihr Hochdosisbereich 17' an den Hochdosisbereich 13' der proximalsten Isoenergieschicht 13 anschließt.

Die Lage der dritt-proximalste Isoenergieschicht kann dann in Abhängigkeit der Lage der zweit-proximalsten Isoenergieschicht 17 bestimmt werden (nicht dargestellt). Diese Bestimmung der Lage findet damit indirekt in Abhängigkeit der Lage der proximalsten Isoenergieschicht 13 statt (über die Lage der zweit-proximalsten Isoenergieschicht 17). Die darauf folgende Isoenergieschicht kann dann in analoger Weise positioniert werden, bis das gesamte Zielvolumen 11 von aufeinander folgenden Isoenergieschichten überdeckt ist.

In dem hier gezeigten Ausführungsbeispiel sind damit alle Lagen derjenigen Isoenergieschichten, die distal zur proximalsten Isoenergieschicht 13 liegen, zumindest indirekt in Abhängigkeit der Lage der proximalsten Isoenergieschicht 13 bestimmt worden.

Dies kann z.B. erfolgen, indem ein regelmäßiger Abstand gewählt wird, der z.B. so gewählt werden kann, dass dann die distalste Isoenergieschicht das Zielvolumen 11 gerade noch abdeckt. Es können auch Verfahren, wie sie in der DE 10 2008 009 765 A1 beschrieben sind, eingesetzt werden.

Fig. 3 zeigt ein Ausführungsbeispiel, bei dem in einem ersten Schritt die Lage der proximalsten 13 und der distalsten Isoenergieschicht 15 geplant werden. Die Lagebestimmung der proximalsten Isoenergieschicht 13 erfolgt wie oben beschrieben.

Die Lagebestimmung der distalsten Isoenergieschicht 15 erfolgt derart, dass die distalste Isoenergieschicht 15 die distale Seite des Zielvolumens 11 gerade noch abdeckt. Dies kann z.B. dadurch geschehen, dass der zugehörige Hochdosisbereich 15' so positioniert wird, dass die distale Kante 19 des Hochdosisbereichs 15' näher an der distalen Stelle des Zielvolumens 11 liegt als die proximale Kante 27 des Hochdosisbereichs 15'.

Die zwischen der proximalsten 13 und der distalsten Isoenergieschicht 15 liegenden Isoenergieschichten 17, 18 können daraufhin selektiert werden, z.B. wie anhand von Fig. 3 beschrieben. In diesem Fall findet die Positionierung der dazwischen liegenden Isoenergieschichten 17, 18 in Abhängigkeit der Lage der proximalsten Isoenergieschicht 13 statt. Bei der zweit-distalsten 18 und der distalsten Isoenergieschicht 15 überlappen sich die Hochdosisbereiche 15', 18' stärker als bei den weiter proximal gelegenen Isoenergieschichten 17.

Die Lagebestimmung zumindest einiger der zwischen der proximalsten und der distalsten Isoenergieschicht angeordneten Isoenergieschichten kann aber auch dadurch erfolgen, dass die Lagebestimmung auch unter Berücksichtigung der Lage der distalsten Isoenergieschicht erfolgt. Auf diese Weise kann ein ggf. notwendiger Überlapp von Hochdosisbereichen zwischen mehreren Isoenergieschichten aufgeteilt werden (hier nicht dargestellt).

Generell sind dabei Aufteilungen zu bevorzugen, bei denen die Hochdosisbereiche in der distalen Hälfte oder im distalen Teil des Zielvolumens 11 stärker überlappen als in der proximalen Hälfte oder im proximalen Teil des Zielvolumens 11. Bei einer Bestrahlung des distaleren Teils des Zielvolumens 11 wird nämlich der proximalere Teils des Zielvolumens 11 bereits mit einer Vordosis belegt, sodass diese Bestrahlung mit der "Vor-Dosis" es ermöglicht macht, einen größeren Schichtabstand zu wählen und damit einhergehend einen geringeren Überlapp an Hochdosisbereichen.

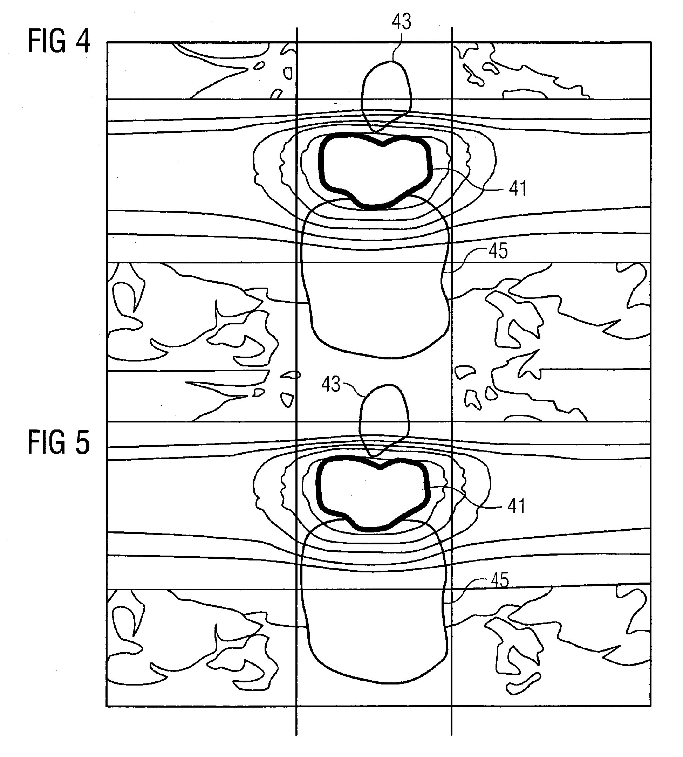

Fig. 4 zeigt an einem Patientenbeispiel eine Dosisverteilung für ein Zielvolumen, die mit einem Bestrahlungsplanungsverfahren nach dem Stand der Technik berechnet worden ist. Fig. 5 zeigt eine Dosisverteilung für dasselbe Zielvolumen, die mit einem Bestrahlungsplanungsverfahren mit proximal adaptiver Positionierung der Isoenergieschichten berechnet worden ist.

Gezeigt ist jeweils eine CT-Abbildung, in der die Prostata 41 als zu bestrahlendes Zielvolumen sowie die Blase 45 und das Rektum 43 als zu schonende Risikoorgane abgebildet sind.

Das Beispiel in Fig. 4 zeigt die Dosisverteilung anhand von Isodosen-Linien für zwei Felder, wobei ein konstantes Raster bzw. ein konstanter Schichtabstand gewählt wurde und die Isoenergieschichten für eine Bestrahlung mit einem 3mm-Ripple-Filter vorgesehen sind.

In Fig. 5 hingegen ist eine Dosisverteilung an gleicher CT-Schichtposition gezeigt für ebenfalls 2 Felder gezeigt, wobei die proximale Isoenergieschicht an das Zielvolumen gezielt adaptiert wurde und die Isoenergieschichten für die Bestrahlung mit einem 4mm-Ripple-Filter (realisierbar durch zwei gekreuzte Ripple-Filter geringerer Tiefe) vorgesehen sind. Zu sehen ist, dass trotz der größeren Energieverbreiterung (und damit des größeren Hochdosisbereichs) eine leicht verbesserte Normalgewebsdosis proximal des Tumors erzielt werden kann. Die beiden senkrechten schwarzen Linien dienen der Orientierung und markieren für das Standardraster die proximale Ausdehnung der 95% Isodose.

Letztlich zeigt das Beispiel die Vermeidung von Dosis im gesunden Gewebe durch die geschickte Wahl und Berücksichtung der proximalen Energieschicht bzw. deren Lage.

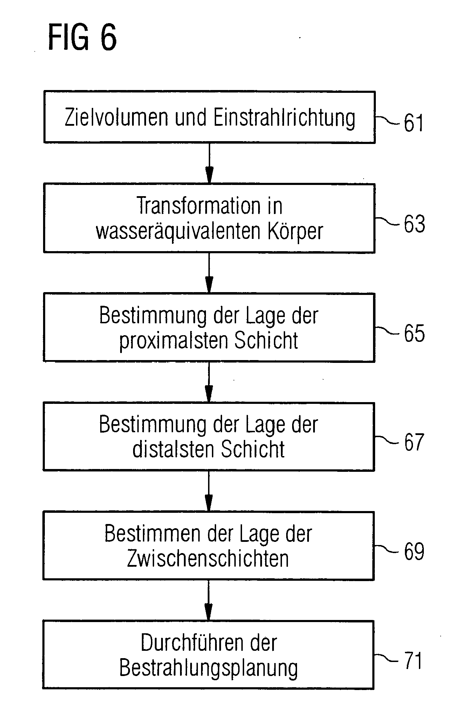

Fig. 6 zeigt ein schematisches Ablaufdiagramm von Verfahrensschritten, die bei einer Ausführungsform des Verfahrens durchgeführt werden können.

In einem ersten Schritt erfolgt die Vorgabe eines Zielvolumens in einem zu bestrahlenden Zielobjekt. Beispielsweise kann ein Zielvolumen in einem Planungs-Computertomographie-Datensatz eingezeichnet werden, z.B. in Interaktion mit einem Anwender oder unter Zuhilfenahme von Segmentierungsverfahren. Nachdem das Zielvolumen bestimmt worden ist, kann eine Einstrahlrichtung festgelegt werden, von der aus das Zielvolumen bestrahlt werden soll (Schritt 61). Die Ausdehnung des Bragg-Peaks für die Bestrahlung einer Isoenergieschicht kann gewählt werden, oder die Dicke einer Isoenergieschicht bzw. die Dicke des Hochdosisbereichs der Isoenergieschicht.

Zudem kann eine Transformation des zu bestrahlenden Zielobjekts in Abhängigkeit der Einstrahl- bzw. Feldrichtung in einen wasseräquivalenten Körper erfolgen (Schritt 63).

Es erfolgt die Bestimmung der Lage der proximalsten Isoenergieschicht in Bezug auf das Zielvolumen (Schritt 65).

Anschließend erfolgt die Bestimmung der Lage der distalsten Isoenergieschicht in Bezug auf das Zielvolumen (Schritt 67).

Zumindest eine weitere Isoenergieschicht, die zwischen der proximalsten und der distalsten liegt, wird anschließend derart positioniert, dass dieses Positionieren in Abhängigkeit der Lage der proximalsten Isoenergieschicht und gegebenenfalls in Abhängigkeit der Lage der distalsten Isoenergieschicht erfolgt (Schritt 69).

Nach erfolgter Schichtpositionierung der das Zielvolumen überdeckenden Schichten kann dann die weitere Bestrahlungsplanung nach bekannten Verfahren erfolgen (Schritt 71). Z.B. können dann diejenigen Rasterpunkte in einer Schicht festgelegt werden, die in einem Scanverfahren zur Bestrahlung angefahren werden sollen. Ebenso kann dann die Dosis bestimmt werden, mit der jede Schicht bestrahlt werden soll. Z.B. kann die genaue Teilchenzahl bestimmt werden, mit der jeder anzufahrende Rasterpunkt der Schicht bestrahlt werden soll, um eine geplante Solldosis im Zielvolumen zu erreichen. Die weitere Bestrahlungsplanung verwendet dabei die Lage der Isoenergieschichten, wie sie zuvor bestimmt worden sind.

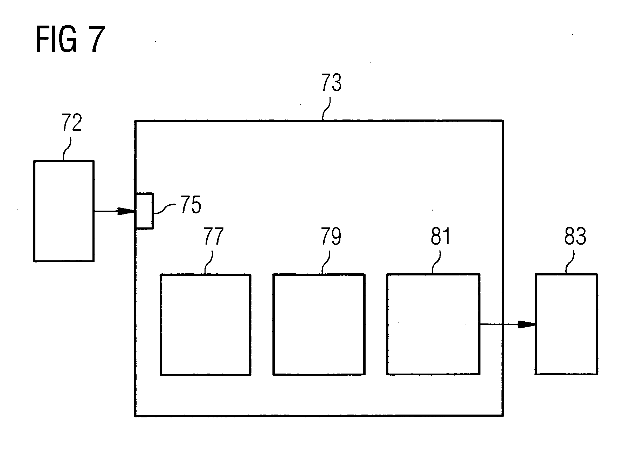

Fig. 7 zeigt eine Darstellung einer Bestrahlungsplanungseinrichtung, mit der ein derartiges Verfahren durchgeführt werden kann. Die Bestrahlungsplanungseinrichtung kann z.B. in eine Rechnereinheit 73 implementiert werden.

Die Rechnereinheit 73 verfügt über eine Schnittstelle 75 zum Laden eines Abbildungsdatensatzes 72, auf dem die folgende Bestrahlungsplanung dann basiert.

Über eine Eingabeeinrichtung 77 kann ein Anwender das zu bestrahlende Zielvolumen in den Abbildungsdatensatz 72 einzeichnen, insbesondere mit Hilfe von Segmentierungsalgorithmen.

Die Rechnereinheit 73 umfasst ferner ein Schichtpositionierungsmodul 79, das derart konfiguriert ist, dass eine erste Position einer proximalsten Isoenergieschicht in Bezug auf das zu bestrahlende Zielvolumen bestimmt wird und dass eine zweite Position zumindest einer weiteren Isoenergieschicht, die distal zu der proximalsten Isoenergieschicht liegt, in Bezug auf das zu bestrahlende Zielvolumen unter Verwendung der ersten Position bestimmt wird. Die Rechnereinheit 73 und insbesondere das Schichtpositionierungsmodul 79 können derart konfiguriert sein, dass Ausführungsformen des Verfahrens wie oben beschrieben, ausgeführt werden können.

Nach erfolgter Schichtpositionierung kann ein Bestrahlungsplanungsmodul 81 einen Bestrahlungsplan 83 basierend auf der Schichtpositionierung erstellen und diesen z.B. in einer Datenbank abspeichern.

## Patentansprüche

1. Verfahren zur Bestrahlungsplanung für ein Partikeltherapie-Bestrahlungsverfahren, in der die zu applizierende Dosis entlang mehrerer in Strahlrichtung hintereinander folgenden Isoenergieschichten (13, 15, 17, 18) zu applizieren ist, umfassend folgende Schritte:
- Vorgeben eines zu bestrahlenden Zielvolumens (11),
- Bestimmen einer ersten Position einer proximalsten Isoenergieschicht (13) in Bezug auf das zu bestrahlende Zielvolumen (11),
- Bestimmen einer zweiten Position zumindest einer weiteren Isoenergieschicht (17, 18), die distal zu der proximalsten Isoenergieschicht (13) liegt, in Bezug auf das zu bestrahlende Zielvolumen (11) unter Verwendung der ersten Position.

2. Verfahren nach Anspruch 1,
wobei die zumindest eine weitere Isoenergieschicht (17, 18) zwischen der proximalsten Isoenergieschicht (13) und einer distalsten Isoenergieschicht (15) liegt, und
wobei das Bestimmen der zweiten Position dadurch erfolgt, dass eine dritte Position der distalsten Isoenergieschicht (15) in Bezug auf das zu bestrahlende Zielvolumen (11) bestimmt wird, und dass die zweite Position unter Verwendung der dritten Position bestimmt wird.

3. Verfahren nach Anspruch 1 oder 2,
wobei beim Bestimmen einer Position einer der Isoenergieschichten (13, 15, 17, 18) ein Hochdosisbereich (13', 15', 17', 18') bestimmt wird, der den zur Isoenergieschicht (13, 15, 17, 18) zugeordneten Bereich mit einer bestimmten Dosisbelegung kennzeichnet, und dass das Bestimmen der Position der Isoenergieschicht (13, 15, 17, 18) unter Verwendung des dieser Isoenergieschicht (13, 15, 17, 18) zugeordneten Hochdosisbereichs (13', 15', 17', 18') erfolgt.

4. Verfahren nach Anspruch 3,
wobei eine Ausdehnung des Hochdosisbereichs (13', 15', 17', 18') bestimmt wird, und zwar in Abhängigkeit von der verwendeten Partikelsorte, von der Anfangsenergie der verwendeten Partikel und/oder Einstellungen der Partikeltherapieanlage, die bei der geplanten Bestrahlung zu verwenden sind.

5. Verfahren nach Ansprüche 3 bis 4,
wobei beim Bestimmen der ersten Position der proximalsten Isoenergieschicht (13) der zugeordnete Hochdosisbereich (13') derart platziert wird, dass dessen proximale Seite (21) näher an der proximalen Seite (25) des Zielvolumens (11) liegt als die distale Seite (23) des Hochdosisbereichs (13`).

6. Verfahren nach einem der Ansprüche 1 bis 5,
wobei eine Umrechnung des Zielvolumens (11) in einen wasseräquivalenten Körper erfolgt und wobei das Bestimmen der ersten Position der proximalsten Isoenergieschicht (13) in Bezug auf das zu bestrahlende Zielvolumen (11) unter Verwendung des umgerechneten Zielvolumens erfolgt.

7. Bestrahlungsplanungseinrichtung zur Bestrahlungsplanung für ein Partikeltherapie-Bestrahlungsverfahren, in der eine zu applizierende Dosis entlang mehrerer in Strahlrichtung hintereinander angeordneten Isoenergieschichten (13, 15, 17, 18) zu applizieren ist,
mit einer Eingabeeinrichtung (77) zum Vorgeben eines zu bestrahlenden Zielvolumens (11),
mit einer Schichtpositionierungseinrichtung (79)
- zum Bestimmen einer ersten Position einer proximalsten Isoenergieschicht (13) in Bezug auf das zu bestrahlende Zielvolumen (11), und
- zum Bestimmen einer zweiten Position zumindest einer weiteren Isoenergieschicht (17, 18), die distal zu der proximalsten Isoenergieschicht (13) liegt, in Bezug auf das zu bestrahlende Zielvolumen (11) unter Verwendung der ersten Position.

8. Bestrahlungsplanungseinrichtung nach Anspruch 7, wobei die Bestrahlungsplanungseinrichtung ausgebildet ist zur Durchführung eines Verfahrens nach einem der Ansprüche 1 bis 6.

## Claims

1. Method for radiation planning for a particle therapy radiation method, in which the dose to be applied is to be applied along a plurality of iso-energy layers (13, 15, 17, 18) following one another in the beam direction, comprising the following steps:
- predefining a target volume (11) to be irradiated,
- determining a first position of a most proximal iso-energy layer (13) in relation to the target volume (11) to be irradiated,
- determining a second position of at least one further iso-energy layer (17, 18), which is situated distally from the most proximal iso-energy layer (13), in relation to the target volume (11) to be irradiated, using the first position.

2. Method according to Claim 1,
wherein the at least one further iso-energy layer (17, 18) lies between the most proximal iso-energy layer (13) and a most distal iso-energy layer (15), and
wherein the second position is determined by virtue of the fact that a third position of the most distal iso-energy layer (15) in relation to the target volume (11) to be irradiated is determined and that the second position is determined using the third position.

3. Method according to Claim 1 or 2,
wherein a high dose region (13', 15', 17', 18') is determined when determining a position of one of the iso-energy layers (13, 15, 17, 18), which high dose region characterises the region assigned to the iso-energy layer (13, 15, 17, 18) with a specific dose application, and in that the position of the iso-energy layer (13, 15, 17, 18) is determined using the high dose region (13', 15', 17', 18') assigned to this iso-energy layer (13, 15, 17, 18).

4. Method according to Claim 3,
wherein an extent of the high dose region (13' , 15', 17' , 18') is determined, to be precise depending on the utilized particle type, on the initial energy of the utilized particles and/or settings of the particle therapy installation, which are to be used during the planned irradiation.

5. Method according to Claims 3 to 4,
wherein the associated high dose region (13') is placed when determining the first position of the most proximal iso-energy layer (13) in such a way that the proximal side (21) thereof lies closer to the proximal side (25) of the target volume (11) than the distal side (23) of the high dose region (13').

6. Method according to one of Claims 1 to 5,
wherein there is a conversion of the target volume (11) into a water-equivalent body and wherein the first position of the most proximal iso-energy layer (13) in relation to the target volume (11) to be irradiated is determined using the converted target volume.

7. Radiation planning apparatus for radiation planning for a particle therapy radiation method, in which a dose to be applied is to be applied along a plurality of iso-energy layers (13, 15, 17, 18) arranged following one another in the beam direction,
with an entry apparatus (77) for predefining a target volume (11) to be irradiated,
with a layer positioning apparatus (79)
- for determining a first position of a most proximal iso-energy layer (13) in relation to the target volume (11) to be irradiated, and
- for determining a second position of at least one further iso-energy layer (17, 18), which is situated distally from the most proximal iso-energy layer (13), in relation to the target volume (11) to be irradiated, using the first position.

8. Radiation planning apparatus according to Claim 7, wherein the radiation planning apparatus is configured to carry out a method according to one of Claims 1 to 6.

## Revendications

1. Procédé de planification d'irradiation pour un procédé d'irradiation pour thérapie par particules, selon lequel la dose à appliquer doit être appliquée le long de plusieurs couches isoénergétiques (13, 15, 17, 18) qui se succèdent dans la direction du faisceau, comprenant les étapes suivantes :
- spécification d'un volume cible à irradier (11) ;
- détermination d'une première position d'une couche isoénergétique la plus proximale (13) par rapport au volume cible à irradier (11) ;
- détermination, par rapport au volume cible à irradier (11), en utilisant la première position, d'une deuxième position au moins d'une autre couche isoénergétique (17, 18) qui est distale par rapport à la couche isoénergétique la plus proximale (13).

2. Procédé selon la revendication 1, l'au moins une autre couche isoénergétique (17, 18) étant située entre la couche isoénergétique la plus proximale (13) et une couche isoénergétique la plus distale (15) et la détermination de la deuxième position s'effectuant par détermination d'une troisième position de la couche isoénergétique la plus distale (15) par rapport au volume cible à irradier (11) et par détermination de la deuxième position en utilisant la troisième position.

3. Procédé selon la revendication 1 ou 2, dans lequel lors de la détermination d'une position de l'une des couches isoénergétiques (13, 15, 17, 18), une région (13', 15', 17', 18') à laquelle est appliquée une dose élevée est déterminée, laquelle caractérise la région associée à la couche isoénergétique (13, 15, 17, 18) par une distribution de dose déterminée, et la détermination de la position de la couche isoénergétique (13, 15, 17, 18) se faisant en utilisant la région (13', 15', 17', 18') à laquelle est appliquée une dose élevée et qui est associée à cette couche isoénergétique (13, 15, 17, 18).

4. Procédé selon la revendication 3, un élargissement de la région (13', 15', 17', 18') à laquelle est appliquée une dose élevée étant déterminé, à savoir en fonction du type de particules utilisé, de l'énergie initiale des particules utilisées et/ou des réglages de l'installation de thérapie par particules qui doivent être utilisés lors de l'irradiation prévue.

5. Procédé selon les revendications 3 à 4, la région associée (13') à laquelle est appliquée une dose élevée étant placée de manière telle, lors de la détermination de la première position de la couche isoénergétique la plus proximale (13), que son côté proximal (21) est plus proche du côté proximal (25) du volume cible (11) que le côté distal (23) de la région (13') à laquelle est appliquée une dose élevée.

6. Procédé selon l'une des revendications 1 à 5, une conversion du volume cible (11) en un corps équivalent en eau étant effectuée et la détermination de la première position de la couche isoénergétique la plus proximale (13) s'effectuant par rapport au volume cible à irradier (11) en utilisant le volume cible converti.

7. Dispositif de planification d'irradiation pour la planification de l'irradiation pour un procédé d'irradiation pour thérapie par particules, dans lequel une dose à appliquer doit être appliquée le long de plusieurs couches isoénergétiques (13, 15, 17, 18) qui se succèdent dans la direction du faisceau, comportant un dispositif de saisie (77) pour spécifier un volume cible à irradier (11),
comportant un dispositif de positionnement de couche (79)
- pour déterminer une première position d'une couche isoénergétique la plus proximale (13) par rapport au volume cible à irradier (11) ;
- pour déterminer, par rapport au volume cible à irradier (11), en utilisant la première position, une deuxième position au moins d'une autre couche isoénergétique (17, 18) qui est distale par rapport à la couche isoénergétique la plus proximale (13).

8. Dispositif de planification d'irradiation selon la revendication 7, le dispositif de planification d'irradiation étant réalisé pour exécuter un procédé selon l'une des revendications 1 à 6.
